# EUROPEAN PATENT APPLICATION

(11) **EP 1 258 225 A2**
(43) Date of publication of application: **20.11.2002**
(21) Application number: 02100458.5
(22) Date of filing: 08.05.2002
(51) Int. Cl.: A61B 17/16

(54) **Tool for making threaded surgical holes**

(30) Priority: 16.05.2001 FI 20011038
(71) Applicant: Inion Ltd., 33520 Tampere (FI)
(72) Inventor: Happonen, Harri, 33823, Tampere (FI); Pohjonen, Timo, 33710, Tampere (FI)
(74) Representative: Kaukonen, Juha Veikko

(57) **Abstract**

A tool for making threaded surgical holes. The tool has a drilling tip (1) that drills the hole and a thread-forming section (2) that forms the thread (9) in the hole and comprises a thread (5) that shapes bone. The height (h) of the thread (5) of the thread-forming section is substantially at least 10% of the outer diameter (D) of the thread-forming section (2).

## Description

The invention relates to a tool for making threaded surgical holes, the tool comprising a drilling tip that drills the hole and a thread-forming section that forms the thread in the hole and comprises a thread that shapes bone, and that is arranged at least for the main part after the drilling tip in the direction of drilling.

In surgery, for instance cranial and facial surgery, fractures are usually fixed by plates that are screwed to the bone. The plate holds the bone in a correct position so as to allow it to heal in the best possible manner. Plates and screws made of titanium are most commonly used, but they are also made of materials that absorb into the organ system, i.e. biodegradable materials. When the plate and screw are made of a material absorbing into the system, they need not be removed from the system, thus avoiding removal surgery after the bone has healed. This is naturally advantageous with respect to patient satisfaction, resource load and costs.

When fixing the plates in place with screws, the screws are arranged through holes made in the plate and the threads of the screws are driven into the fixation hole drilled into the bone. The threads of titanium screws are typically self-cutting, i.e. the screw cuts the threads to the bone. Instead, screws made of absorbing materials must be driven into a threaded hole. This is typically done by first drilling a hole into the bone with a drill, after which threads are made into the hole with a screw tap. Making a threaded hole in this manner requires many steps and is slow. Combinations of a drill and a screw tap, or self-tapping drills, are also known. They comprise a drilling tip and a threading section. In other words, the hole and its threads are made at the same time with the same rotating motion. This speeds up the making of the hole essentially.

As known, bone comprises a hard and dense surface layer, i.e. cortex, and a porous inner layer. In this respect, self-tapping drills include a problem: they are only suited for mono-cortical fixation in soft bones, i.e. for drillings done through only one, quite thin cortical layer. An example of such mono-cortical fixation is maxillary operations, because when making a threaded hole, first the cortex on the drill side is penetrated and after that, the operation continues in the porous inner layer. Bi-cortical holes, i.e. holes that extend through two cortical layers, need instead to be made in two steps by first drilling and then making the thread with a screw tap. This is due to the fact that when the tip of a prior-art self-tapping threading tool meets the hard cortex on the opposite side of the bone in relation to the drill side, it is very probable that the already made thread breaks and collapses under a sudden and high axial stress. Making a hole into the thick and hard cortex is also very difficult, because axial stresses easily break the thread already made into the hole. This is why the thick and hard cortex is drilled in the conventional manner in two steps by first drilling and then threading with a screw tap. As mentioned earlier, two-step hole making is slow and difficult.

It is an object of this invention to provide a novel and improved tool for making threaded surgical holes.

The tool of the invention is characterized in that the height of the thread of the thread-forming section is substantially at least 10% of the outer diameter of the thread-forming section.

The essential idea of the invention is that the height h of the thread is at least 10%, preferably at most 25% and most preferably 12 to 15% of the outer diameter D of the thread-forming section. Further, the idea of a preferred embodiment of the invention is that the thread-forming section forms the thread of the hole partly by cutting and partly by compressing bone.

The tool of the invention provides the advantage that the thread of the thread-forming section is so high that it is possible to make not only mono-cortical holes, but also bi-cortical holes and holes in the thick and hard cortex quickly, reliably and without risk.

The invention is described in more detail in the attached drawings, in which
Figure 1 is a schematic perspective view of a tool of the invention,
Figure 2 is a schematic side view of one end of the tool of Figure 1, and
Figure 3 is a schematic cross-sectional side view of a threaded hole made with the tool of the invention and having a screw inserted in it.

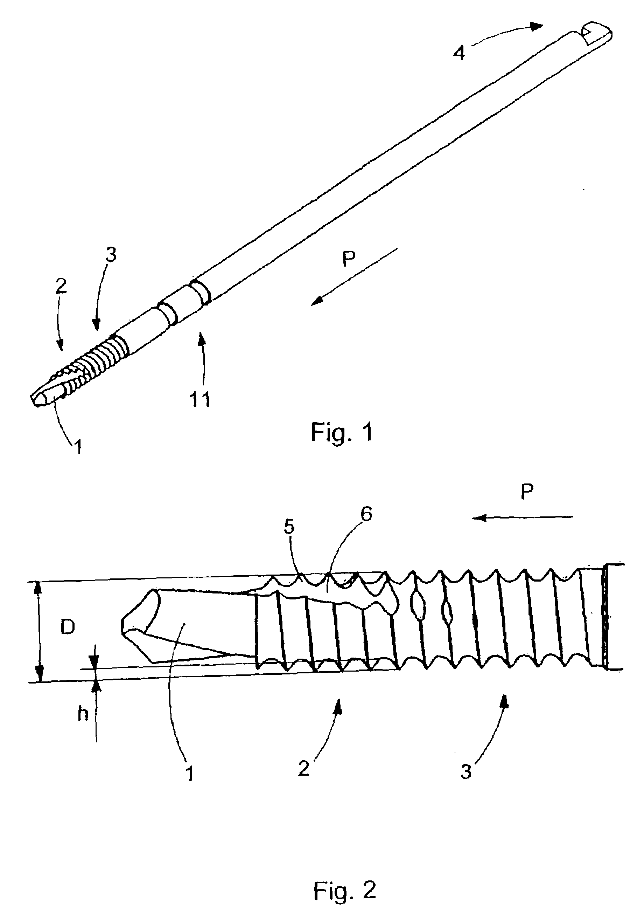

Figure 1 is a schematic perspective view of a tool of the invention. The tool is an essentially round bar in basic shape, one end of which has the means for making a threaded hole, i.e. a drilling tip 1, and a thread-forming section 2 arranged after it in the drilling direction P and a guiding thread section 3. The opposite end of the tool has an element 4 for fastening the tool to a wrenching means, such as a drill with a low rotational frequency or a manual handle, with which the tool is rotated around its axis to make the hole. The tool has a code 11, such as a colour code, by which the tool can be identified.

The tool is preferably made of (stainless) steel. The means for making the threaded hole are described in greater detail in Figure 2.

Figure 2 is a schematic side view of the means for making a threaded hole of the tool of Figure 1. The drilling tip 1 is a drill bit known per se. The drilling tip 1 penetrates first the bone being drilled and makes a blank hole. A thread-forming section 2 is arranged after the drilling tip 1. The thread-forming section 2 comprises a thread 5 that forms a thread in to the blank hole made by the drilling tip 1 into the bone. It should be noted that the bone is not shown in the figures. In the embodiment of Figures 1 and 2, the thread-forming section 2 only begins after the drilling tip 1, but in a second embodiment of the invention, the thread-forming section 2 already begins from the drilling tip. The thread-forming section 2 forms the thread in the bone by cutting and/or compressing. A cut thread is more permanent in shape. Bone, especially its soft inner layer, is namely to some extent elastic. If the thread is formed solely by compressing the bone, the bone may revert back to its original shape relatively quickly and the measurements of the thread change so that the screw intended for the hole no longer fits in. The reversion may even take place immediately when the tool is removed from the hole. On the other hand, since a compressed thread tries to revert to the original shape, it locks a screw fitted into the hole very tightly in place. By suitably combining the cutting and compressing characteristics of the thread-forming section 2, it is possible to make a threaded hole, to which a screw can be easily driven and which on the long run locks the screw tightly into place.

Bone chips cut by the drilling tip 1 and thread-forming section 2 rise up from the hole along a chip groove 6. A guiding thread section 4 is arranged after the thread-forming section 2. It does not essentially shape the hole, but supports the tool so as to make its handling and the making of the hole easy.

The thread 5 of the thread-forming section is shaped such that the axial bearing area of the thread formed in the bone is larger than usual or customary. This is accomplished in such a manner that the height h of the thread 5 of the thread-forming section is substantially 10 to 25%, most preferably 12 to 15% of the outer diameter D of the thread-forming section. A large bearing area reduces essentially the stresses directed to the threads of the hole that occur in bi-cortical drilling, for instance, when the drilling tip meets the cortex on the opposing side of the bone in relation to the drilling side. The thus generated stress is mainly directed to the threads in the cortex on the drilling side, but to a minor extent also to the threads in the porous inner layer of the bone. With the tool of the invention, it is possible to safely make bi-cortical drillings and threaded holes in any bone. If the height of the thread is more than 25%, the axial rigidity of the tool is so low that it is difficult to make a hole especially in the very hard cortex. In most cases the axial rigidity of the tool and the safe making of bi-cortical drillings are well balanced when the height h is substantially 12 to 15% of the outer diameter D of the thread-forming section.

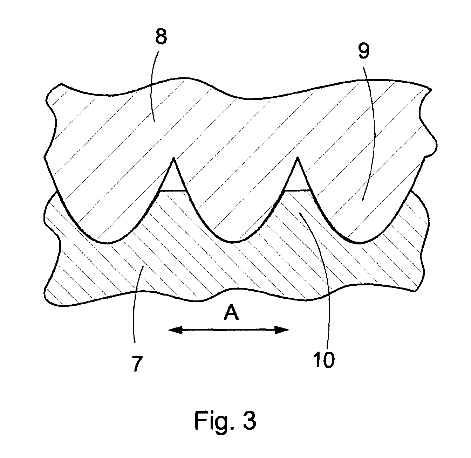

Figure 3 is a schematic cross-sectional side view of a threaded hole made with the tool of the invention and having a screw inserted in it. First a threaded hole is made with the tool and then the tool is rotated out of the hole in the opposite direction to the drilling direction P. After this, a screw 7 is inserted into the hole. As can be seen, the thread 9 made by the tool into the bone 8 is quite substantially higher than the thread 10 of a conventional screw 7 known per se. In other words, the height h of the thread 5 of the thread-forming section is oversized with respect to the thread 10 of the screw 7. The large surface area of the high thread 9 in the axial direction A of the hole reduces essentially the axial A stresses of the hole generated in the bone, whereby the thread 9 formed into the bone does not break easily.

The drawings and the related description are only intended to illustrate the idea of the invention. The invention may vary in detail within the scope to the claims. Thus, the thread-forming section 2 can form the thread 9 only by cutting the bone 7 or only by compressing the bone 7. The tool can be implemented without the guiding thread part 3. The shape and pitch of the thread 5 of the thread-forming section and the number of threads may differ from what is shown in the figures.

## Claims

1. A tool for making threaded surgical holes, which tool comprises a drilling tip (1) that drills the hole and a thread-forming section (2) that forms the thread in the hole and comprises a thread (5) that shapes bone and that is arranged at least for the main part after the drilling tip (1) in the drilling direction, **characterized in that** the height (h) of the thread (5) of the thread-forming section is substantially at least 10% of the outer diameter (D) of the thread-forming section (2).

2. A tool as claimed in claim 1, **characterized in that** the height (h) of the thread (5) is substantially at most 25% of the outer diameter (D) of the thread-forming section (2).

3. A tool as claimed in claim 1 or 2, **characterized in that** the height (h) of the thread (5) is substantially 12 to 15% of the outer diameter (D) of the thread-forming section (2).

4. A tool as claimed in any one of the preceding claims, **characterized in that** it comprises a guiding thread part (3) that is arranged after the thread-forming section (2) in the drilling direction.

5. A tool as claimed in any one of the preceding claims, **characterized in that** the thread-forming section (2) forms the thread (9) in the hole by cutting the bone (8).

6. A tool as claimed in any one of claims 1 to 4, **characterized in that** the thread-forming section (2) forms the thread (9) in the hole by compressing the bone (8).

7. A tool as claimed in any one of claims 1 to 4, **characterized in that** the thread-forming section (2) forms the thread (9) in the hole partly by cutting and partly by compressing the bone (8).
